# EUROPEAN PATENT SPECIFICATION

(11) **EP 1 114 151 B1**
(45) Date of publication and mention of the grant of the patent: **29.06.2011**
(21) Application number: 99944181.9
(22) Date of filing: 15.09.1999
(51) Int. Cl.: C12N 15/12, C07K 14/705, C07K 14/72, A61K 38/04, A61P 15/06, G01N 33/50, G01N 33/68

(54) **G PROTEIN-COUPLED RECEPTOR ANTAGONISTS**
G-PROTEIN-GEKOPPELTE REZEPTOR-ANTAGONISTEN
ANTAGONISTES DU RECEPTEUR COUPLE LA PROTEINE G

(30) Priority: 17.09.1998 US 154627
(43) Date of publication of application: 11.07.2001
(73) Proprietor: Hopital Sainte-Justine, Montreal, Quebec H3T 1C5 (CA)
(72) Inventor: CHEMTOB, Sylvain, Montréal, Québec H4W 1G1 (CA); PERI, Krishna, G., Montréal, Québec H3H 1Y3 (CA)
(74) Representative: Holliday, Louise Caroline
(86) International application number: PCT/CA1999/000844
(87) International publication number: WO 2000/017348

(56) References cited:
- WO-A-93/09104
- WO-A-95/00551
- WO-A-96/23225
- US-A- 5 688 938
- KITANAKA ET AL.: "Phloretin as an antagonist of prostaglandin F2alpha receptor in cultured rat astrocytes" JOURNAL OF NEUROCHEMISTRY, vol. 60, no. 2, February 1993 (1993-02), pages 704-708, XP000876615
- SUGIMOTO ET AL.: "Failure of parturition in mice lacking the prostaglandin f receptor" SCIENCE, vol. 277, 1 August 1997 (1997-08-01), pages 681-683, XP002130206 cited in the application
- GRIFFIN B W ET AL: "AL-8810: a novel prostaglandin F2 alpha analog with selective antagonist effects at the prostaglandin F2 alpha ( FP ) receptor." JOURNAL OF PHARMACOLOGY AND EXPERIMENTAL THERAPEUTICS, (1999 SEP) 290 (3) 1278-84. , XP000876534

### SEQUENCE LISTING

<110> HÔPITAL SAINTE-JUSTINE
   CHEMTOB, Sylvain
   PERI, Krishna G.
<120> G PROTEIN-COUPLED RECEPTOR ANTAGONISTS
<130> 12667-16PCT FC/ld
<150> US 09/154,627
   <151> 1998-09-17
<160> 12
<170> FastSEQ for Windows Version 3.0
<210> 1
   <211> 8
   <212> PRT
   <213> FP receptor peptides
<400> 1
   Ile Leu Gly His Arg Asp Tyr Lys
    1               5
<210> 2
   <211> 8
   <212> PRT
   <213> FP receptor peptides
<400> 2
   Trp Glu Asp Arg Phe Tyr Leu Leu
    1               5
<210> 3
   <211> 8
   <212> PRT
   <213> FP receptor peptides
<400> 3
   Tyr Gln Asp Arg Phe Tyr Leu Leu
    1               5
<210> 4
   <211> 8
   <212> PRT
   <213> FP receptor peptides
<400> 4
   Ile Leu Ala His Arg Asp Tyr Lys
    1               5
<210> 5
   <211> 8
   <212> PRT
   <213> FP receptor peptides
<400> 5
   Ile Leu Gly Phe Arg Asp Tyr Lys
   1 5
<210> 6
   <211> 8
   <212> PRT
   <213> FP receptor peptides
<400> 6
   Ile Leu Gly His Lys Asp Tyr Lys
   1 5
<210> 7
   <211> 8
   <212> PRT
   <213> FP receptor peptides
<400> 7
   Ile Leu Gly His Arg Asn Tyr Lys
   1 5
<210> 8
   <211> 8
   <212> PRT
   <213> FP receptor peptides
<400> 8
   Ile Leu Gly His Gln Asp Tyr Lys
   1 5
<210> 9
   <211> 7
   <212> PRT
   <213> FP receptor peptides
<400> 9
   Ile Leu Gly His Arg Asp Tyr
   1 5
<210> 10
   <211> 8
   <212> PRT
   <213> FP receptor peptides
<400> 10
   Ile Leu Gly Trp Arg Asp Tyr Lys
   1 5
<210> 11
   <211> 8
   <212> PRT
   <213> FP receptor peptides
<400> 11
   Ile Leu Gly Xaa Arg Asp Tyr Lys
    1               5
<210> 12
   <211> 8
   <212> PRT
   <213> FP receptor peptides
<400> 12
   Ser Asn Val Leu Cys Ser Ile Phe
    1               5

## Description

### BACKGROUND OF THE INVENTION

### (a) Field of the Invention

The invention relates to development of antagonists of a G protein-coupled receptor, which Wind to the G protein-coupled receptor from the extracellular side in a manner different from that of the natural ligand.

### (b) Description of Prior Art

prostaglandins are derived from the oxygenation of arachidonic acid by prostaglandin synthetases. Prostaglandins mediate a wide variety of physiological actions, such as vasomotricity, sleep/wake cycle, intestinal secretion, lipolysis, glomelular filtration, mast cell degranulation, neurotransmission, platelet aggregation, leuteolysis, myometrial contraction and labor, inflammation and arthritis, patent ductus arteriosus, cell growth and differentiation (Coleman, R.A. et al., 1994, Pharmacol. Rev. 46:205-229; Goetzl, E.J. et al., 1995, FASEB J. 9:1051-10585). Prostanoids mediate their actions through binding to distinct receptors, which belong to the super family of rhodopsin-like seven transmembrane helical receptors. These receptors are coupled to heterotrimeric G-proteins comprising of α, β and γ subunits which, upon activation, elicit alternations in cell calcium, initiate phosphoinositide hydrolysis or promotion or repression of cyclic adenosine monophosphate synthesis (Strader C. D. et al., 1994, Ann. Rev. Biochem. 63:101-132).

Of the five pharmacologically distinct prostanoid receptors for PGE₂, PGI₂, PGD₂, PGF_{2α} and TxA₂ and their many isoforms, the receptor for PGF_{2α}, also called FP receptor, shows limited tissue distribution, predominantly expressed in corpora leutea, uterine myometrium, trabecular meshwork of the eye, and to a lesser extent in vascular smooth muscle. Initiation of labor is marked by tremendous rise in PGF_{2α} levels and increased uterine contractility. The wide spread use of PGF_{2α} analogues to induce labor in veterinary industry points to the primary role of PGF_{2α} and its receptor in parturition. This is underscored by the fact that mice lacking the FP receptor fail to undergo labor (Sugimoto et al., 1997, Science 277:81-83). In face of escalating costs incurred as a result of premature births and associated complications to the neonate, such as intraventricular hemorrhage, bronchopulmonary displasia and periventricular leukomalacia leading to cerebral palsy, prolongation of gestation by arresting premature labor is an effective preventive therapy. The relative success of nonsteroidal anti-inflammatory drugs as a short-term therapy toward prevention of premature labor is based on their inhibitory actions upon the synthesis of prostaglandins, particularly PGE₂ and PGF_{2α}. However, inhibition of PGE₂ is associated with serious complications to the fetus such as the closure of ductus arteriosus, renal failure and pulmonary hypertension.

At another level, PGF_{2α} has been attributed a major role in dysmenorrhea, a condition which afflicts 5%-7% of premenopausal women. A pre-menstrual increase in PGF_{2α} levels resulting in myometrial spasms underlies the pathogenesis of this disorder. Lack of effective antagonists of FP receptor for extended therapy hampered the advances in preventing premature labor and associated sequelae, and the design of such antagonists is the subject of this application.

Human FP receptor is a 45 kDa integral membrane glycoprotein, consisting of 359 amino acids and shares only 47% sequence identity with EP₁ receptor, and to a lesser extent with other prostanoid receptors (Abramovitz et al., 1994, J. Biol. Chem. 269:2632-2636). Binding of PGF_{2α} to FP receptor is followed by the activation of G_{αqβγ} complex, increased GTP binding by the G_{αq} subunit, stimulation of phospholipase cβ activity, release of inositol phosphates, increased intra-cellular calcium and subsequent signal transduction phenomena ultimately leading to smooth muscle contraction (Coleman, R.A. et al., 1994, Pharmacol. Rev. 46:205-229). The FP receptor is the only efficacious target for development of therapeutic drugs since a few G_{α}-proteins catalyze the actions of hundreds of G-protein coupled receptors, thus targets downstream from the receptor are essentially of little use.

Antagonists of FP receptors directed to the ligand binding site could be of limited use since ligand based inhibitors show cross reactivity with other prostanoid receptors. Their efficacy will be compromised in face of tremendous increase in PGF_{2α} concentrations in myometrium at the onset of labor and in menstruation. The high basal activity of the receptors in the absence of ligand limits the use of ligand-based inhibitors.

It would be highly desirable to be provided with antagonists of FP receptors, which do not crossreact with other prostanoid receptors, and are effective even in the presence of excess ligand.

Other relevant prior art includes (1) WHO 95/00551; (2) WO 96/23225; (3) WO 93/09104; (4) US 5688938; (5) Kitanaka J et al., J Neurochem., 1993 Feb; 60(2): 704-8, PMID: 809348; (6) Sugimoto Y et al., Science, 1997 Aug 1; 277(5326): 681-3, PMID: 9235889; and (7) Griffin BW et al., J Pharmacol Exp Ther., 1999 Sep; 290(3): 1278.84, PMID: 10454504.

### SUMMARY OF THE INVENTION

one aim of the present invention is to provide antagonists of FP receptors, which do not crossreact with other prostanoid preceptors.

Another aim of the present invention is to provide inhibitors of FP receptors by a novel strategy to target the extracellular domains of the receptor protein.

According to the present invention there is provided a prostaglandin FP receptor antagonist, which comprises an amino acid sequence of the prostaglandin FP receptor selected from the group consisting of: ilghrdyk (PCP-8; SEQ ID NO:1); wedrfyll (PCP-10; SEQ ID NO:2); YQDRFYLL (PCP-14; SEQ ID NO:3); ILGHRDYK (PCP-13; SEQ ID NO:13); ILAHRDYK (PCP-13.7; SEQ ID NO:4); ILaHRDYK (PCP13.8; SEQ ID NO:14); ILGFRDYK (PCP-13.11; SEQ ID NO:5); ILGHKDYK (PCP-13.13; SEQ ID NO:6); ILGHRNYK (PCP-13.14; SEQ ID NO:7); ILGHQDYK (PCP-13.18; SEQ ID NO:8); ILGHRDY-amide (PCP-13.20; SEQ ID NO:9); ILGHRDYK-amide (PCP-13.21; SEQ ID NO:15); ILGWRDYK (PCP-13.22; SEQ ID NO:10); SNVLCSIF (PCP-15; SEQ ID NO:12) and ILGXRDYK (PCP-13.24; SEQ ID NO:11), wherein X is cyclohexyl alanine, wherein small letters indicate L-amino acids and capital letters indicate D-amino acids.

Also provided according to the present invention is a peptide having prostaglandin FP receptor antagonist activity, said peptide consisting of a variant sequence of SEQ ID NO: 1 or SEQ ID NO: 2 in which one or two amino acid residues are substituted or deleted, wherein said variant sequence contains L- and/or D-amino acids, and wherein the C-terminal group of said peptide is a CO₂H group or a CONH₂ group.

Also provided according to the present invention is the prostaglandin FP receptor antagonist or the peptide of the present invention, for use in preventing premature delivery of a fetus.

Preferably, the antagonist or peptide in-use specifically binds to the extracellular face of the receptor, thereby hampering uterine contractions.

Also provided according to the present invention is the prostaglandin FP receptor antagonist or the peptide of the present invention, for use in preventing and/or treating dysmenorrhea.

Preferably, the antagonist or peptide in-use specifically binds to the extracellular face of the receptor to hamper transduction of a signal thereby reducing the pain associated with contractions.

Also provided according to the present invention is a pharmaceutical composition comprising at least one prostaglandin FP receptor antagonist or at least one peptide according to the present invention, in association with a pharmaceutically acceptable carrier.

Also provided according to the present invention is a method of identifying a compound as a prostaglandin FP receptor antagonist or the peptide according to the present invention capable of binding to the extracellular elements of said receptor, comprising the steps of:
a) culturing cells which express said receptor or identifying animal tissues *ex vivo* where physiological consequences are dependent on said receptor;
b) contacting said cells or tissues with said compound at a concentration of 10⁻¹⁰ to 10⁻³ M to be tested for antagonist activity at said receptor; and
c) measuring a response to alter the transduction of a signal resulting in physiological consequences selected from the group consisting of increments in cell calcium, phosphoinositide hydrolysis, cell growth and/or differentiation, altered gene expression, and smooth muscle contraction or dilation, wherein said response is indicative of antagonist activity.

Also provided according to the present invention is a method for determining activity of the antagonist or the peptide of the present invention as a prostaglandin FP receptor antagonist capable of binding to the extracellular elements of the said receptor, comprising the steps of:
a) culturing cells which express said receptor or identifying animal tissues *ex vivo* where physiological consequences are dependent on said receptor;
b) contacting said cells or tissues with said antagonist or peptide at a concentration of 10⁻¹⁰ to 10⁻³ M to be tested for antagonist activity at said receptor; and
c) measuring a response to alter the transduction of a signal resulting in physiological consequences selected from the group consisting of increments in cell calcium, phosphoinositide hydrolysis, cell growth and/or differentiation, altered gene expression, and smooth muscle contraction or dilation.

Also provided according to the present invention is the use of a therapeutically effective amount of the prostaglandin FP receptor antagonist or the peptide of the present invention, for the preparation of a medicament for preventing premature delivery of a fetus.

Also provided according to the present invention is the use of a therapeutically effective amount of the prostaglandin FP receptor antagonist or the peptide of the present invention, for the preparation of a medicament for preventing and/or treating dysmenorrhea.

For the purpose of the present invention the following terms are defined below.

The expression "a G protein-coupled receptor antagonist" is intended to mean any natural or synthetic compound, peptide protein, antibody, peptidomimetic or small chemical molecules, without limitation, insofar as it can specifically bind to the extracellular structural elements of the G protein-coupled receptor to alter transduction of a signal. More preferably, the antagonist does not crossreact with other prostanoid receptors.

The expression "functional derivatives" of G protein-coupled receptor antagonist is intended to mean mimetic compounds and/or structurally unrelated compounds with respect to G protein-coupled receptor antagonist, which can also specifically bind to the extracellular structural elements of the G protein-coupled receptor to alter transduction of a signal.

The expression "peptidomimetic thereof" is intended to mean any chemical entities, mimetic compounds and/or structurally unrelated compounds with respect to a G protein-coupled receptor antagonist, which can also specifically bind to the extracellular structural elements of the G protein-coupled receptor to alter transduction of a signal.

### BRIEF DESCRIPTION OF THE DRAWINGS

Fig. 1 illustrates the inhibitory effects of PCP-8 and PCP-10 on FP receptor function upon stimulation with PGF_{2α} in accordance with the embodiment of the present invention;
Fig. 2A illustrates the effects of PCP-8 and PCP-10 on the diameter of the microvessels of pig retina upon stimulation with either PGF_{2α} or thromboxane A₂ mimetic, U46619;
Fig. 2B illustrates the dose response of PGF_{2α} on the diameter of pig microvessels treated previously with PCP-8 or PCP-10;
Fig. 2C illustrates the effects of thromboxane A₂ mimetic, U46619, on the diameter of pig microvessels treated previously with PCP-8 and PCP-10;
Fig. 3A illustrates the effects of PCP-10 upon spontaneous contractions of uterine smooth muscle;
Fig. 3B illustrates the dose response of prostaglandin F₂. in the presence/absence of PCP-8 and_PCP-10 upon uterine smooth muscle contraction; and
Fig. 4 illustrates the reversal of basal tone of bovine myometrium even in the presence of FP receptor ligand, PGF_{2α}.

### DETAILED DESCRIPTION OF THE INVENTION

In accordance with the present invention, there is provided a new class of G protein-coupled receptor antagonists, which bind to the extracellular molecular surface, thus hamper signal transduction.

Also provided is a novel strategy to target the extracellular loops of the receptor which contribute to the structural or functional integrity of the receptor. Antagonists thus bind to cognate elements in the extracellular surface of the receptor and prevent the receptor function by interfering with its signal initiation or transduction.

There is provided proof of selectivity of the antagonists to FP receptor by showing an absence of their effects on a related prostanoid receptor for thromboxane A₂, known as TP receptor which is also involved in smooth muscle contraction.

### Preparation of inhibitors

### Chemical synthesis of PCP-8 and PCP-10:

All peptides which are 8 amino acids in length were synthesized using F-moc chemistry and solid phase Merrifield method two peptides, PCP-8 and PCP-10. These peptides were purified by HPLC and their purity tested by mass spectroscopy.

In accordance with the present invention, a novel strategy of using peptides derived from the extracellular domains of prostaglandin F_{2α} receptor, FP, to inhibit the signal transduction and the functional consequences of FP receptor. This method could be generalized to all G protein-coupled receptors. Peptides derived from the first and second extracellular loops of FP receptor were found to be effective inhibitors of FP receptor.

The present invention could be readily understood by referring to the following examples, which are given to illustrate the invention rather than to limit its scope.

### EXAMPLE I

### Effects of peptides, PCP-8 and PCP-10, on ligand-induced phosphoinositide hydrolysis in mammalian cells overexpressing the FP receptor

Both PCP-8 and -10 were tested in HEK293 cells expressing the human FP receptor. For this purpose, HEK 293 cells stably expressing human FP receptor were plated in 12-well plates in DMEM medium containing 10% fetal bovine serum, penicillin (10 U/ml) and streptomycin (10 µg/ml) and cultured in a humidified atmosphere containing 5% CO₂ at 37°C. After the wells were 80% confluent, the cells were labeled with 2 µCi/ml of [³H]-*myo* inositol overnight. Next day, the cells were washed once with PBS, and incubated in 0.5 ml of Kreb's buffer containing 10 mM LiCl and indicated concentrations of PCP peptides for 30 min. PGF_{2α} at 1µM was added to the cells and the incubation was carried out for an additional 30 min. The cells were solubilized with 0.1 N NaOH for 10 min and neutralized with 0.1 N formic acid. The lysates were collected and 1 ml each of methanol and chloroform were sequentially added and vortexed briefly. After centrifugation to separate the phases, inositol phosphates were separated by ion exchange chromatography as described below (Berridge, M.J. et al., 1983, Biochem. J. 212:473-482).

Briefly, the medium was discarded and the IP3 synthesis was stopped by adding 0.6 ml ice-cold methanol. The cells were scraped and collected into polypropylene tubes. Distilled water (0.5 ml) and chloroform (0.6 ml) were added and vigorously vortexed for 2 min. The phases were separated by centrifugation at 6000 x g for 10 min. The aqueous phase was applied to AG-1X-8™ (Formate form) anion exchange columns (1 ml bed volume) and free inositol was eluted with 10 ml of water, followed by 60 mM ammonium formate in 0.1 M formic acid. Then, the inositol phosphates were eluted with 5 ml of 1.2 M ammonium formate in 0.1 M formic acid. After adding 3 volumes of scintillation cocktail (Optiphase-HiSafe III), the eluates were counted by scintillation spectrophotometry.

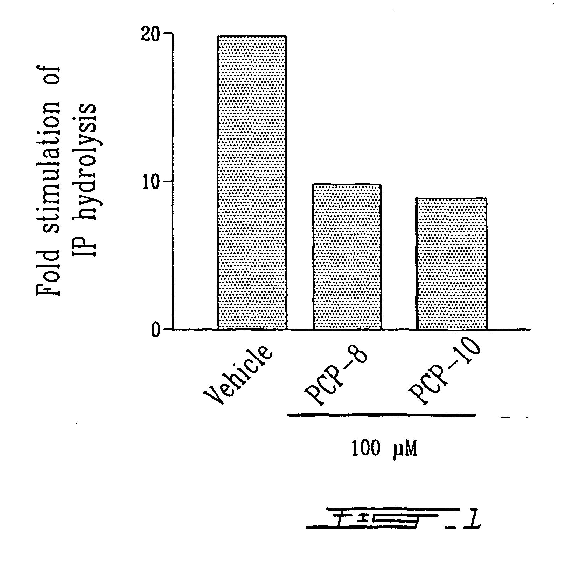

The results of these experiments are shown in Fig. 1. Data are expressed as fold stimulation of inositol phosphate synthesis by 1 µM PGF_{2α} compared to the unstimulated controls. Both PCP-8 and -10 at 100 µM potently inhibited inositol phosphate synthesis initiated by the action of PGF_{2α} on FP receptor. The half maximal inhibitory concentrations for both PCP-8 and - 10 were slightly less than 100 µM.

### EXAMPLE II

### Testing PCP peptides in porcine eyecup model of ex vivo vasomotricity assay

In order to see if the peptides could inhibit FP function using an *ex vivo* model, we chose porcine eyecup model, an *ex vivo* assay of vascular constriction in porcine retinas which we previously described and validated (Li et al., 1996 J. Pharmacol. Expt. Therapeut. 278: 370-377; Li et al., 1997 Am. J. Physiol. 273: R1283-90; Abran et al., 1997 Am. J. Physiol. 272: R995-1001). Since FP receptor densities in newborn vasculature are minimal due to down regulation by high levels of circulating prostaglandins in the perinatal period, the newborn pigs were treated with a prostaglandin synthetase blocker, ibuprofen (30 mg/Kg of bodyweight/ 8 h for 24 h) to increase the density of the receptors as well as their vasomotor effects. By inhibiting circulating prostaglandins; we were able to show potent inhibition of FP receptor-mediated second messenger synthesis as well as FP-mediated vascular constriction in this eyecup model.

To prepare eyecups, a circular incision was made 3-4 mm posterior to ora serrata to remove the interior segment and vitreous body with minimal handling of the retina. The remaining eyecup was fixed with pins to a wax base in a 20 ml tissue bath containing 20 ml of Kreb's buffer (pH 7.35-7.45), protease inhibitors, leupetin and aprotinin (10 µg/ml each), and equilibrated with 21% oxygen and 5% carbon dioxide at 37°C. The preparations were allowed to stabilize for 30 min. Peptides at 100 µM were added and incubation was continued for 30 min before the addition of PGF_{2α}.

Cumulative concentration-responses of PGF_{2α} and TxA₂ mimetic, U46619, (10⁻¹⁰ to 10⁻⁵ M) curves were constructed separately. To assess the reversibility of the antagonists, the eyecups were thoroughly washed (which would wash away the peptide) with incubation medium and concentration response curves for PGF_{2α} were determined. The outer vessel diameter was recorded with a video camera mounted on a dissecting microscope (Zeiss M 400™) and the responses were quantified by a digital image analyzer (Sigma Scan Software, Jandel Scientific, Corte Madera, CA). Vascular diameter was recorded before and 10 min following the topical application of the agent. Each measurement was repeated three times and showed <1% variability.

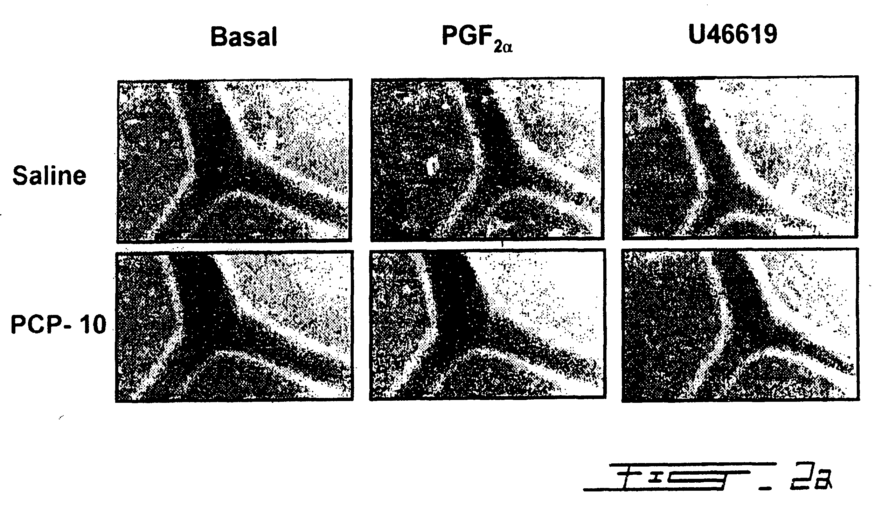

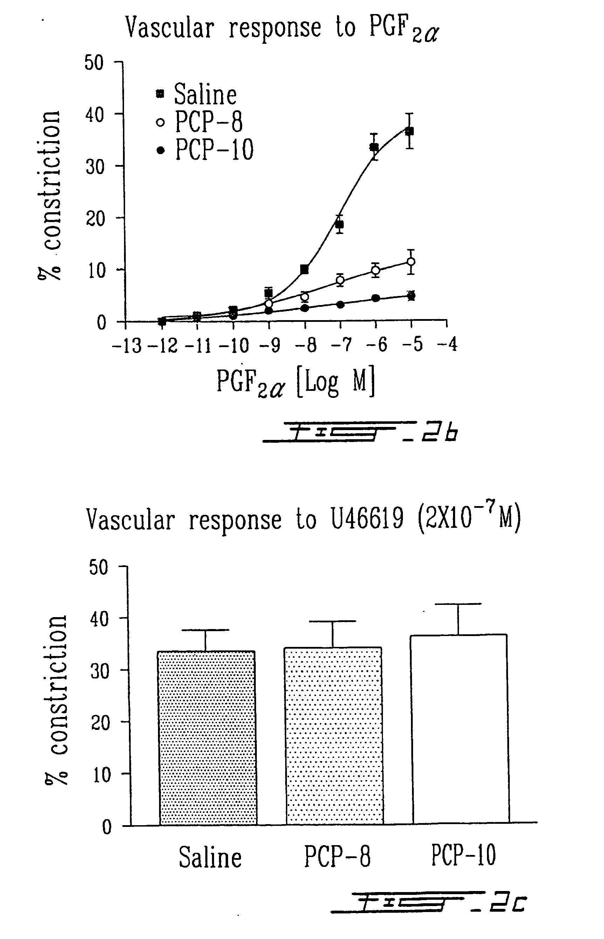

The results are shown in Fig. 2. The peptide PCP-10 had no effect on the basal tone (diameter of the microvessel) of the vessel (Fig. 2A; left panels). Addition of 1 µM of PGF_{2α} potently constricted the vessel in the absence of the peptide (middle-top panel), whereas presence of PCP-10 at 100 µM markedly inhibited PGF_{2α}-mediated vasoconstriction (middle-bottom panel). The peptide had no effect on the vasoconstriction effected by 1 µM TxA₂ mimetic, U46619, (right panels) acting on another prostanoid receptor coupled to constriction, namely TP receptor. Similar results were obtained for PCP-8 as well. A dose response of PGF_{2α} on the vascular diameter in the presence/absence of PCP-8 and PCP-10 peptides are presented in Fig. 2B. Both peptides abrogated the vasomotor responses even at concentrations exceeding 1µM of PGF_{2α}, suggesting, as expected, that the peptides may be acting in a non-competitive fashion. However, the peptides had no effect on vasoconstriction produced by thromboxane A₂ (Fig. 2C).

Similarly, a peptide derived from the first extracellular loop of FP receptor, PCP-15, inhibited PGF_{2α}-induced constriction (10⁻⁷M) (88.1% over untreated control; Table 1).

### EXAMPLE III

### Testing peptide variants of PCP-8 in porcine eyecup model of ex vivo vasomotricity assay

In order to understand the structural requirements of PCP-8 in its inhibitory action on PGF_{2α}-induced vasoconstriction, different amino acids in PCP-8 sequence were replaced with other D- or L- amino acids and the resulting peptides were chemically synthesized and tested in porcine eyecup model of *ex vivo* vasomotricity assay. These peptide variants are listed in Table 1.

**Table 1**

| **Amino acid sequences of peptide variants of PCP-8 and their inhibitory potency in porcine eyecup model of *ex vivo* vasomotricity assay** | | | | |
|---|---|---|---|---|
| **Peptide PCP-** | **%Vasomotor response (of constriction)¹** | **% inhibition of maximal max. response²** | **Peptide sequence** | **SEQ ID NO:** |
| 8 | 50.0 | 50.0 | ilghrdyk | 1 |
| 10 | 20.0 | 80.0 | wedrfyll | 2 |
| 14 | 36.0 | 64.0 | YQDRFYLL | 3 |
| 13 | 20.0 | 80.0 | ILGHRDYK | 13 |
| 13.7 | 23.8 | 76.2 | ILAHRDYK | 4 |
| 13.8 | 46.8 | 53.2 | ILaHRDYK | 14 |
| 13.11 | 13.0 | 87.0 | **ILGFRDYK** | 5 |
| 13.13 | 36.9 | 63.1 | ILGNKDYK | 6 |
| 13.14 | 40.3 | 59.7 | ILGHRNYK | 7 |
| 13.18 | 30.0 | 70.0 | ILGHQDYK | 8 |
| 13.20 | 49.6 | 50.4 | ILGHRDY-amide | 9 |
| 13.21 | 46.2 | 53.8 | ILGHRDYK-amide | 15 |
| 13.22 | 16.6 | 83.4 | ILGWRDYK | 10 |
| 13.24 | 6.2 | 93.8 | ILGXRDYK | 11 |
| 15 | 11.9 | 88.1 | SNVLCSIF | 12 |

| | | | | |
|---|---|---|---|---|
| ¹Percent vasomotor response in the presence of 100 µM peptide is calculated as percent change in average vascular diameter produced by 10⁻⁷ M PGF_{2α} to the eyecup in the presence of the peptide compared to maximal constriction observed in the absence of the peptide. ²Percent inhibition produced by each peptide is calculated as (100-per cent vasomotor response). | | | | |

Small letters indicate L-amino acids and capital letters indicate D- amino acids. I = isoleucine; L= leucine; G =glycine; H=histidine, R=Arginine; D=Aspartic acid; Y=Tyrosine; X-Lysine; A-Alanine; W= Tryptophan; E-Glutamic acid; F= Phenyl alanine; Q=Glutamine; N=Aspargine; P=Proline; S=Serine; X=Cyclohexyl alanine. Peptides were dissolved in DMSO freshly just before the experiment as 10 mM stocks and added to the eye cups 30 min before the addition of 10⁻⁷ M PGF_{2α}.

A total of 25 variants of PCP-8 were synthesized and the efficacious or potent peptides are listed in Table 1. These peptides incorporate L- to D-amino acid changes, deletions, subtle variations in aromaticity, hydrogen bond donor status as opposed to ionic interactions and hydrophobicity. These peptides were tested at 100 µM concentration in porcine retinal vasomotricity assay and the results are summarized in Table 1.
The results are summarized as follows:
1. Converting all L-amino acids of PCP-8 to D-amino acids (PCP-13) increased the inhibitory potency dramatically. Removal of N-terminal hydrophobic dipeptide sequence from either PCP-8 (PCP-11) or PCP-10 (PCP-12) resulted in significant reduction in the inhibitory action of the peptides.
2. Glycine to alanine (13.7) does not change the activity of PCP-13, whereas change to proline (13.16), L-alanine (13.8), or deletion of the residue (13.17) entirely resulted in loss of activity. Glycine is an important linker residue separating the HRD motif from the IL hydrophobic sequence.
3. HRD-motif is important for the activity of PCP-13. Alanine substitutions (13.1-13.3) or to change to L-configuration (13.4-13.6) resulted loss of inhibitory activity of PCP-13. Aromaticity of His is more important than the positive charge, since H to F (13.11) or W (13.22) or X (13.24), but not to Y (13.23), did not result in significant reduction of peptide inhibitory potency. Side chain length appears to be more critical in case of D residue than R; changing D to E (13.12) resulted in loss of half of the inhibitory activity whereas R to K (13.13) or to Q (13.18) affected the activity of PCP-13 moderately. D to N (13.14) resulted in moderate loss of activity, whereas a serine substitution (13.19) lead to drastic loss of activity of PCP-13.
4. Deletion of terminal lysine (13.15) or substitution with W (13.9) resulted in complete loss of activity; however, conversion of terminal carboxylate into an amide (13.20 & 13.21) resulted in moderate gain of activity of the peptide inhibitor. Substitution of aromatic residue, Y, with E (13.10) completely abolished the inhibitory potency of PCP-13.

Thus the structure of PCP-13 in D-configuration appears to consists of a N-terminal hydrophobic anchor spaced from the central HRD motif by a glycine residue possibly resulting in a turn conformation of the active peptide; Aromatic and hydrophobic interactions at the carboxy terminus may also add to the potency of PCP-13.

### EXAMPLE IV

### Testing PCP peptides in porcine uterine strip of ex vivo basal contraction assay

In *ex vivo* experiments using porcine uterine strips, the peptides were able to prevent both basal and PGF_{2α}-induced contraction.

Uterine tissues from non-pregnant adult pigs were obtained from a local slaughter house and transported to the laboratory on ice. Uterine myometrial strips of approximately 1 cm in length were set up in organ baths containing Kreb's buffer equilibrated with 21% oxygen at 37°C as we have described (Potvin, W. et al., 1990, Br. J. Pharmacol. 100:341-347; Varma, D.R. and Chemtob, S., 1993, J. Pharmacol. Expt. Ther. 265:1096-1104). Contractions were recorded by force transducers on Grass-polygraph. Strips were incubated with or without 100 µM peptides for 30 min before adding PGF_{2α} in step-wise increments (10⁻⁹ to 10⁻⁶ M). Data were expressed as percentage increase over the basal level of average tension (g).

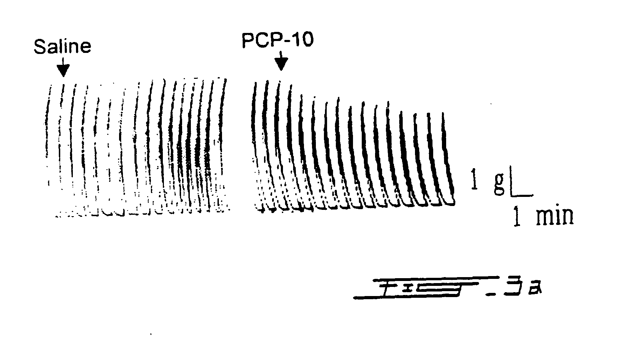

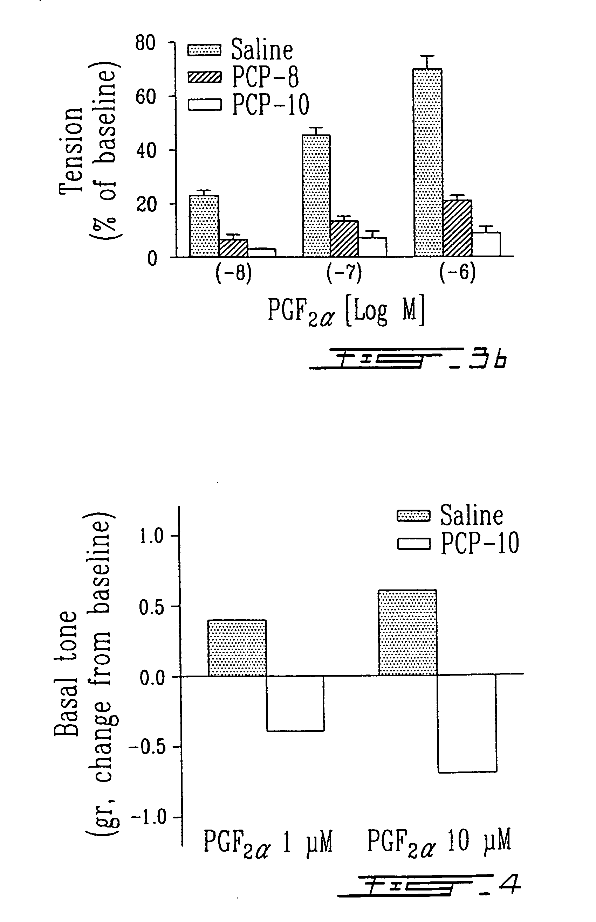

A graph of spontaneous uterine contractions (known to be dependent upon prostanoids, mainly PGF_{2α}) in the absence and the presence of 100 µM PCP-8 are shown in Fig. 3A. Addition of peptide to the strips reduced the force of basal contraction. A dose response of PGF_{2α} on uterine contractility in the presence or absence of PCP-8 and PCP-10 peptides is shown in Fig. 3B. More than 60% (PCP-8) and 80% (PCP-10) reduction in uterine contraction was observed in all concentrations of PGF_{2α} tested. Thus, both these peptides reduced spontaneous as well as PGF_{2α}-induced contractions in the uterine strips.

### EXAMPLE V

### Testing PCP peptides in bovine uterine strip of ex vivo basal contraction assay

Uterine tissues from non-pregnant adult bovine animals were obtained from a local slaughter house and transported to the laboratory on ice. Uterine myometrial strips of approximately 1 cm in length were set up in organ baths containing Kreb's buffer equilibrated with 21% oxygen at 37°C as described above. Contractions were recorded on Grass-polygraph by force transducers. Strips were incubated with or without 100 µM peptides before adding PGF_{2α} in step-wise increments (10⁻⁸ to 10⁻⁶ M). Data were expressed as change in basal level of average tension (g). The results are shown in Fig. 4. Application of PCP-10 peptide at 100 µM reversed the basal tone (contractile state) of the uterine muscle. Addition of PGF_{2α} up to 10µM did not affect the relaxation produced by PCP-10 suggesting that the effects of PCP peptides are independent of the ligand, which was also shown in the previous results.

While the invention has been described in connection with specific embodiment thereof, it will be understood that it is capable of further modifications and this application is intended to cover any variations, uses or adaptations of the invention following in general, the principles of the invention and including such departures from the present disclosure as come within the known customary practice within the art to which the invention pertains and as may be applied to the essential features hereinbefore set forth, and as follows in the scope of the appended claims.

## Claims

1. A prostaglandin FP receptor antagonist , which comprises an amino acid sequence of the prostaglandin FP receptor selected from the group consisting of: ilghrdyk (PCP-8; SEQ ID NO:1); wedrfyll (PCP-10; SEQ ID NO:2); YQDRFYLL (PCP-14; SEQ ID NO:3); ILGHRDYK (PCP-13; SEQ ID NO:13); ILAHRDYK (PCP-13.7; SEQ ID NO:4); ILaHRDYK PCP13.8; SEQ ID NO:14); ILGFRDYK (PCP-13.11; SEQ ID NO:5); ILGHKDYK (PCP-13.13; SEQ ID NO:6); ILGHRNYK (FCP-13.14; SEQ ID NO:7); ILGHQDYK (PCP-13.18; SEQ ID NO:8); ILGHRDY-amide (PCP-13.20; SEQ ID NO:9); ILGHRDYK-amide (PCP-13.21; SEQ ID NO:15); ILGWRDYK (PCP-13.22; SEQ ID NO:10); SNVLCSIF (PCP-15; SEQ ID NO:12) and ILGXRDYK (PCP-13.24; SEQ ID NO:11), wherein X is cyclohexyl alanine, wherein small letters indicate L-amino acids and capital letters indicate D-amino acids.

2. A peptide having prostaglandin FP receptor antagonist activity, said peptide consisting of a variant sequence of SEQ ID NO: 1 or SEQ ID NO: 2 in which one or two amino acid residues are substituted or deleted, wherein said variant sequence contains L- and/or D-amino acids, and wherein the C-terminal group of said peptide is a CO₂H group or a CONH₂ group.

3. The prostaglandin FP receptor antagonist of claim 1 or the peptide of claim 2, for use in preventing premature delivery of a fetus.

4. The prostaglandin FP receptor antagonist of claim 1 or the peptide of claim 2, for use in preventing and/or treating dysmenorrhea.

5. A pharmaceutical composition comprising at least one prostaglandin FP receptor antagonist of claim 1 or at least one peptide of claim 2, in association with a pharmaceutically acceptable carrier.

6. A method of identifying a compound as a prostaglandin FP receptor antagonist of claim 1 or the peptide of claim 2 capable of binding to the extracellular elements of said receptor, comprising the steps of:
a) culturing cells which express said receptor or identifying animal tissues *ex vivo* where physiological consequences are dependent on said receptor;
b) contacting said cells or tissues with said compound at a concentration of 10' ¹⁰ to 10⁻³ M to be tested for antagonist activity at said receptor; and
c) measuring a response to alter the transduction of a signal resulting in physiological consequences selected from the group consisting of increments in cell calcium, phosphoinositide hydrolysis, cell growth and/or differentiation, altered gene expression, and smooth muscle contraction or dilation, wherein said response is indicative of antagonist activity.

7. A method for determining activity of the antagonist of claim 1 or the peptide of claim 2 as a prostaglandin FP receptor antagonist capable of binding to the extracellular elements of the said receptor, comprising the steps of:
a) culturing cells which express said receptor or identifying animal tissues *ex vivo* where physiological consequences are dependent on said receptor;
b) contacting said cells or tissues with said antagonist or peptide at a concentration of 10⁻¹⁰ to 10⁻³ M to be tested for antagonist activity at said receptor; and
c) measuring a response to alter the transduction of a signal resulting in physiological consequences selected from the group consisting of increments in cell calcium, phosphoinositide hydrolysis, cell growth and/or differentiation, altered gene expression, and smooth muscle contraction or dilation.

8. Use of a therapeutically effective amount of the prostaglandin FP receptor antagonist of claim 1 or the peptide of claim 2, for the preparation of a medicament for preventing premature delivery of a fetus.

9. Use of a therapeutically effective amount of the prostaglandin FP receptor antagonist of claim 1 or the peptide of claim 2, for the preparation of a medicament for preventing and/or treating dysmenorrhea.

## Patentansprüche

1. Prostaglandin-FP-Rezeptor-Antagonist, welcher eine Aminosäuresequenz des Prostaglandin-FP-Rezeptors umfasst, die ausgewählt ist aus der Gruppe bestehend aus: ilghrdyk (PCP-8; SEQ ID NO: 1), wedrfyll (PCP-10; SEQ ID NO: 2), YQDRFYLL (PCP-14, SEQ ID NO: 3), ILGHRDYK (PCP-13; SEQ ID NO:13), ILAHRDYK (PCP-13.7, SEQ ID NO: 4), ILaHRDYK (PCP 13.8; SEQ ID NO: 14), ILGFRDYK (PCP-13.11; SEQ ID NO: 5; ILGHKDYK (PCP-13.13; SEQ ID NO: 6), ILGHRNYK (PCP-13.14; SEQ ID NO: 7), ILGHQDYK (PCP-13.18; SEQ ID NO: 8), ILGHRDY-Amid (PCP-13.20; SEQ ID NO:9), ILGHRDYK-Amid (PCP-13.21; SEQ ID NO: 15), ILGWRDYK (PCP-13.22; SEQ ID NO: 10), SNVLCSIF (PCP-15; SEQ ID NO:12) und ILGXRDYK (PCP-13.24; SEQ ID NO: 11), wobei X Cyclohexylalanin ist, wobei Kleinbuchstaben L-Aminosäuren anzeigen und Großbuchstaben D-Aminosäuren.

2. Peptid, welches Prostaglandin-FP-Rezeptor-Antagonist-Aktivität aufweist, wobei das Peptid aus einer Sequenzvariante von SEQ ID NO: 1 oder SEQ ID NO: 2 besteht, in welcher eine oder zwei Aminosäurereste substituiert oder deletiert werden, wobei die Sequenzvariante L- und/oder D-Aminosäuren enthält, und wobei die C-terminale Gruppe des Peptids eine CO₂H-Gruppe oder eine CONH₂-Gruppe ist.

3. Prostaglandin-FP-Rezeptor-Antagonist nach Anspruch 1 oder Peptid nach Anspruch 2 für die Verwendung bei der Vorbeugung der Frühgeburt eines Fötus.

4. Prostaglandin-FP-Rezeptor-Antagonist nach Anspruch 1 oder Peptid nach Anspruch 2 für die Verwendung bei der Vorbeugung und/oder Behandlung von Dysmenorrhö.

5. Pharmazeutische Zusammensetzung, die zumindest einen Prostaglandin-FP-Rezeptor-Antagonisten nach Anspruch 1 oder zumindest ein Peptid nach Anspruch 2 aufweist, in Verbindung mit einem pharmazeutisch verträglichen Träger.

6. Verfahren zur Identifizierung einer Verbindung als Prostaglandin-FP-Rezeptor-Antagonist nach Anspruch 1 oder als Peptid nach Anspruch 2, umfassend die Stufen:
a) Kultivieren von Zellen, die den Rezeptor exprimieren, oder Identifizieren von tierischen Geweben *ex vivo,* bei denen physiologische Auswirkungen von dem Rezeptor abhängig sind,
b) Inkontaktbringen der Zellen oder Gewebe mit der auf Antagonistenaktivität an dem Rezeptor zu testenden Verbindung bei einer Konzentration von 10⁻¹⁰ bis 10⁻³ M, und
c) Messen einer Reaktion, die Transduktion eines Signals zu ändern, was in physiologischen Auswirkungen resultiert, die ausgewählt sind aus der Gruppe bestehend aus Zunahme des Zellcalciums, Phosphoinositid-Hydrolyse, Zellwachstums und/oder - differenzierung, veränderte Genexpression und Kontraktion oder Dilatation der glatten Muskulatur, wobei diese Reaktion ein Zeichen für Antagonistenaktivität ist.

7. Verfahren zur Bestimmung der Aktivität des Antagonisten nach Anspruch 1 oder des Peptids nach Anspruch 2 als Prostaglandin-FP-Rezeptor-Antagonist, der an die extrazellulären Elemente des Rezeptors binden kann, welches die folgenden Stufen umfasst:
a) Kultivieren von Zellen, die den Rezeptor exprimieren, oder Identifizieren von tierischen Geweben *ex vivo,* bei denen physiologische Auswirkungen von dem Rezeptor abhängig sind,
b) Inkontaktbringen der Zellen oder Gewebe mit dem auf Antagonistenaktivität an dem Rezeptor zu testenden Antagonisten oder Peptid bei einer Konzentration von 10⁻¹⁰ bis 10⁻³M, und
c) Messen einer Reaktion, die Transduktion eines Signals zu ändern, was in physiologischen Auswirkungen resultiert, die ausgewählt sind aus der Gruppe bestehend aus Zunahme des Zellcalciums, Phosphoinositid-Hydrolyse, Zellwachstums und/oder - diffierenzierung, veränderte Genexpression und Kontraktion oder Dilatation der glatten Muskulatur, wobei diese Reaktion ein Zeichen für Antagonistenaktivität ist.

8. Verwendung einer therapeutisch wirksamen Menge des Prostaglandin-FP-Rezeptor-Antagonisten nach Anspruch 1 oder des Peptids nach Anspruch 2 für die Herstellung eines Medikaments zur Vorbeugung der Frühgeburt eines Fötus.

9. Verwendung einer therapeutisch wirksamen Menge des Prostaglandin-FP-Rezeptor-Antagonisten nach Anspruch 1 oder des Peptids nach Anspruch 2 für die Herstellung eines Medikaments zur Vorbeugung und/oder Behandlung von Dysmenorrhö.

## Revendications

1. Antagoniste de récepteur FP de prostaglandine, comprenant une séquence d'acide aminé du récepteur FP de prostaglandine choisie dans le groupe constitué de : ilghrdyk (PCP-8 ; SEQ ID N°1) ; wedrfyll (PCP-10, SEQ ID N°2) ; YQDRFYLL (PCP-14, SEQ ID N°3), ILGHRDYK (PCP-13, SEQ ID N°13) ; ILAHRDYK (PCP-13.7, SEQ ID N°4) ; ILaHRDYK (PCP13.8 ; SEQ ID N°14) ; ILGFRDYK (PCP-13.11, SEQ ID N°5) ; ILGHKDYK (PCP-13.13 ; SEQ ID N°6) ; ILGHRNYK (PCP-13.14, SEQ ID N°7) ; ILGHQDYK (PCP-13.18 ; SEQ ID N°8) ; ILGHRDY-amide (PCP-13.20 ; SEQ ID N°9) ; ILGHRDYK-amide (PCP-13.21 ; SEQ ID N°15) ; ILGWRDYK (PCP-13.22 ; SEQ ID N°10) ; SNVLCSIF (PCP-15 ; SEQ ID N°12) et ILGXRDYK (PCP-13.24 ; SEQ ID N°11), dans lequel X est une cyclohexyl-alanine, et dans lequel les lettres en minuscule indiquent des acides aminés L et les lettres en majuscule indiquent des acides aminés D.

2. Peptide ayant une activité antagoniste de récepteur FP de prostaglandine, ledit peptide étant constitué d'une séquence variante de SEQ ID N°1 ou SEQ ID N°2, dans laquelle un ou deux résidus d'acide aminé sont substitués ou éliminés, dans lequel ladite séquence variante contient des acides aminés L et/ou D, et dans lequel le groupe à l'extrémité C-terminale dudit peptide est un groupe CO₂H ou un groupe CONH₂.

3. Antagoniste de récepteur FP de prostaglandine selon la revendication 1 ou peptide selon la revendication 2, pour utilisation dans la prévention d'un accouchement prématuré.

4. Antagoniste de récepteur FP de prostaglandine selon la revendication 1 ou peptide selon la revendication 2, pour utilisation dans la prévention et/ou le traitement de la dysménorrhée.

5. Composition pharmaceutique comprenant au moins un antagoniste de récepteur FP de prostaglandine selon la revendication 1, ou au moins un peptide selon la revendication 2, en association avec un excipient pharmaceutiquement acceptable.

6. Procédé d'identification d'un composé comme un antagoniste de récepteur FP de prostaglandine selon la revendication 1 ou comme un peptide selon la revendication 2, capable de se lier aux éléments extracellulaires dudit récepteur, comprenant les étapes consistant à :
a) cultiver des cellules qui expriment ledit récepteur ou identifier des tissus animaux *ex vivo* où les conséquences physiologiques dépendent dudit récepteur ;
b) mettre lesdites cellules ou lesdits tissus en contact avec ledit composé à une concentration de 10⁻¹⁰ à 10⁻³ M à tester pour l'activité antagoniste audit récepteur ; et
c) mesurer une réponse pour altérer la transduction d'un signal donnant lieu à des conséquences physiologiques choisies dans le groupe constitué d'incréments du calcium cellulaire, de l'hydrolyse de phosphoinositide, de la croissance et/ou de la différenciation cellulaire, de l'expression génique altérée, et de la contraction ou de la dilatation du muscle lisse, dans lequel la réponse est indicative de l'activité antagoniste.

7. Procédé de détermination de l'activité de l'antagoniste selon la revendication 1 ou du peptide selon la revendication 2, comme un antagoniste de récepteur FP de prostaglandine capable de se lier aux éléments extracellulaires dudit récepteur, comprenant les étapes consistant à :
a) cultiver des cellules qui expriment ledit récepteur ou identifier des tissus animaux *ex vivo* où les conséquences physiologiques dépendent dudit récepteur ;
b) mettre lesdites cellules ou lesdits tissus en contact avec ledit antagoniste ou peptide à une concentration de 10⁻¹⁰ à 10⁻³ M à tester pour l'activité antagoniste audit récepteur ; et
c) mesurer une réponse pour altérer la transduction d'un signal donnant lieu à des conséquences physiologiques choisies dans le groupe constitué d'incréments de calcium cellulaire, d'hydrolyse de phosphoinositide, de croissance et/ou de différenciation cellulaire, d'expression génique altérée, et de contraction ou de dilatation du muscle lisse.

8. Utilisation d'une quantité thérapeutiquement efficace de l'antagoniste de récepteur FP de prostaglandine selon la revendication 1 ou du peptide selon la revendication 2, pour la préparation d'un médicament visant à prévenir un accouchement prématuré.

9. Utilisation d'une quantité thérapeutiquement efficace de l'antagoniste de récepteur FP de prostaglandine selon la revendication 1 ou du peptide selon la revendication 2, pour la préparation d'un médicament visant à prévenir et/ou à traiter la dysménorrhée.
